# EUROPÄISCHE PATENTSCHRIFT

(11) **EP 0 771 783 B1**
(45) Veröffentlichungstag und Bekanntmachung des Hinweises auf die Patenterteilung: **05.04.2000**
(21) Anmeldenummer: 96116527.1
(22) Anmeldetag: 16.10.1996
(51) Int. Cl.: C07C 201/00, B01J 19/26, B01J 19/24, C07C 201/08, C07C 205/06, C07C 205/12

(54) **Verfahren zur Aromatennitrierung**
Process for the nitration of aromatics
Procédé pour la nitration de composés aromatiques

(30) Priorität: 22.10.1995 DE 19539205
(43) Veröffentlichungstag der Anmeldung: 07.05.1997
(73) Patentinhaber: JOSEF MEISSNER GMBH & CO, D-50968 Köln (DE)
(72) Erfinder: Gebauer, Jürgen, 53840 Troisdorf (DE); Hermann, Heinrich, Dr., 50858 Köln (DE)
(74) Vertreter: Eggert, Hans-Gunther, Dr.

(56) Entgegenhaltungen:
- EP-A- 0 436 443
- FR-A- 2 695 840
- US-A- 2 512 587
- US-A- 2 737 522

## Beschreibung

Die vorliegende Erfindung betrifft ein kontinuierliches Verfahren zum Nitrieren von einkernigen Aromaten mit einem Gemisch aus Salpetersäure und Schwefelsäure im Zweiphasensystem.

Die Nitrierung von Aromaten wie Benzol, Toluol, Chlorbenzolen etc. zu den entsprechenden Mononitro- bzw. Dinitroderivaten wird technisch vorwiegend kontinuierlich durch Zusammenmischen eines Gemisches aus Salpetersäure und Schwefelsäure (Mischsäure) mit den zu nitrierenden Aromaten durchgeführt. Die für die Nitrierung verwendeten Mischsäuren haben im allgemeinen eine Zusammensetzung von 20 - 30 % Salpetersäure, 55 - 65 % Schwefelsäure und 5 - 25 % Wasser und werden so an die Eigenschaften des zu nitrierenden Aromaten so angepaßt, daß eine Abfallsäure mit ca. 70 % Schwefelsäure und 29 - 30 % Wasser und 0 - 1 % Salpetersäure erhalten wird. Das Mengenverhältnis von zu nitrierendem Aromat und Mischsäure wird stets so gewählt, daß die Nitrierung nicht in homogener Phase, sondern in einem zweiphasigen Gemisch aus Nitriersäure und einer flüssigen Organphase aus Produkt und zu nitrierenden Aromaten erfolgt. Nach einer vom hergestellten Nitroaromaten abhängigen Verweilzeit wird die zweiphasige Nitrieremulsion geschieden und das Produkt und die verbrauchte Mischsäure (Abfallsäure) werden jeweils entweder einer Reinigung (Produkt und Abfallsäure) oder einer Aufkonzentrierung (Abfallsäure) zugeführt.

Die Nitrierung erfolgt im wesentlichen in der Säurephase, d.h., der zu nitrierende Aromat muß aus der Organphase in die Säurephase diffundieren, wo er mit der dort im Überschuß vorhandenen Salpetersäure zum gewünschten Nitroaromaten reagiert.

Der Umsatz pro Zeiteinheit hängt damit nicht nur von den kinetischen Reaktionsparametern ab, sondern auch davon, wie gut der Übergang (Massentransfer) des zu nitrierenden Stoffes aus der Organphase in die Säurephase erfolgt.

Die Austauschfläche zwischen organischer und Säurephase wird normalerweise durch intensives Rühren erzeugt. In der dabei gebildeten Emulsion wird je nach Phasenverhältnis und zu nitrierendem Aromat eine Verteilung der Organphase in der Säurephase oder Säurephase in der Organphase erzeugt.

Bei kontinuierlichen Nitrierungen wird meist in einer Rührkesselkaskade mit vollständiger Rückvermischung gearbeitet, um die vollständige Umsetzung des zu nitrierenden Produktes oder der eingesetzten Salpetersäure zu erreichen.

Bei dieser Arbeitsweise erfolgt die Nitrierung nicht wie im Rohrreaktor mit sich ändernder Umsetzungsgeschwindigkeit in einem Gemisch von Schwefelsäure und Salpetersäure, dessen Konzentrationen sich im Verlaufe der Nitrierung mit steigendem Umsatz über einen weiten Konzentrationsbereich ändert, sondern stets bei konstanter Säurestärke und Salpetersäurekonzentration und dadurch konstanter Umsetzungsgeschwindigkeit.

Die Säurekonzentrationen in der Mischsäure bzw. in den einzelnen Stufen der Rührkesselkaskade wird so gewählt, daß nur der gewünschte Nitrokörper (z.B. Nitrobenzol aus Benzol oder Mononitrotoluol aus Toluol) möglichst schnell gebildet wird, aber eine Weiterreaktion zu den entsprechenden Dinitrokörpern möglichst langsam erfolgt, d.h., daß die Selektivität der gewünschten Umsetzung sehr hoch ist. Aus diesem Grunde wird z.B. die Nitrierung von Benzol und Toluol bei Schwefelsäurekonzentrationen von ca. 70 % in der Nitriersäure und bei möglichst niedriger Temperatur (z.B. Benzol 55°C, Toluol 35 °C) durchgeführt. Wird z.B. die Nitrierung bei höheren Temperaturen z.B. bei Benzol bei Temperaturen um 120 - 135 °C wie z.B. bei einer adiabatischen Nitrierung (US-A-4 091 042, "Adiabatische Herstellung von Nitrobenzol") durchgeführt, muß die Schwefelsäurekonzentration in der Nitriersäure z.B. der Rührkesselkaskade soweit abgesenkt werden, daß die Selektivität bei vorgegebener Verweilzeit im Reaktor erhalten bleibt (z.B. 65 % Schwefelsäure).

Andere Faktoren neben der Acidität der Nitriersäure und der Temperatur, die die Selektivität beeinflussen, sind natürlich die Konzentration des Reaktionspartners Salpetersäure und die Verweilzeit des Gemisches aus Organphase und Säurephase im Reaktor.

Erfolgt die Nitrierung in einem Rohrreaktor durch Zusammenmischen einer Mischsäure mit hoher Salpetersäure- und Schwefelsäurekonzentration und niedrigem Wassergehalt mit dem zu nitrierenden Stoff, ist die Selektivität für das gewünschte Produkt zu Beginn der Umsetzung, niedrig, vor allem bei nicht optimaler, homogener Vermischung der Reaktionspartner, da mit typischen Mischsäuren zu Beginn einer Nitrierung im Rohrreaktor bei erst beginnendem Umsatz durch die starke Abhängigkeit der Nitriergeschwindigkeit von der Acidität des Nitriergemisches (K. Schofield in "Aromatic Nitration", Cambrigde University Press 1980, Cambridge) auch das gebildete Produkt schnell weiternitriert wird (z.B. Mononitrobenzol zu Dinitrobenzol). Dieser Verlust an Selektivität läßt sich nur vermeiden, wenn entweder mit Mischsäuren gearbeitet wird, deren Acidität im Vergleich zu optimalen Nitriersäuren nur geringfügig erhöht ist, so daß eine weitere Reaktion des zuerst gebildeten Produktes noch sehr langsam ist, oder wenn durch schnellen Umsatz in Gegenwart eines Überschusses an zu nitrierenden Aromaten gearbeitet wird, so daß durch den schnellen Verbrauch an Salpetersäure und die Bildung von Wasser die Acidität im Nitriergemisch schnell abgesenkt wird und damit die Selektivität erhalten bleibt.

Der Einsatz von Standardmischtechniken, wie z.B. Mischen in einem Rührkessel oder das Einspritzen des zu nitrierenden Stoffes in einen turbulenten Säurestrahl, wie in einem Injektor, wie erfolgreich für die Nitrierung von Alkoholen wie Glycerin oder Glycol (DE-PS-1 039 049) eingesetzt, wird vor allem beim Einsatz in großtechnischen Anlagen, wo große Massenströme pro Zeiteinheit gemischt werden müssen, als nicht ausreichend betrachtet, um die erforderliche hochdisperse Verteilung des zu nitrierenden Gutes in der Nitriersäure zu erreichen (US-A-4 973 770).

Um dieses Ziel - Erhalten der Selektivität beim Nitrieren im Rohreaktor und minimale Bildung von Nebenprodukten (z.B. bei der Nitrierung von Benzol zu Mononitrobenzol die Bildung von Dinitrobenzol, Nitrophenolen und anderen Oxidationsprodukten) - zu erreichen, wurde versucht, den zu nitrierenden Stoff in der Säurephase möglichst schnell in Form einer homogenen, hochdispersen Emulsion zu verteilen. Dies kann erreicht werden durch Zusammenmischen der Reaktionspartner in einer Kreiselpumpe (US-A-3 092 671) oder durch den Einsatz spezieller Düsen (US-A-4 973 770) oder von einer besonderen Anordnung einer größeren Anzahl von Jet-Mischern (US-A-4 994 242 und US-A-5 313 009).

Durch die schnelle Erzeugung einer großen Phasengrenzfläche zwischen Säure- und Organphase soll ein Maximum an Transfer des zu nitrierenden Stoffes wie z.B. Benzol aus der Organphase in die Säurephase, in der die Umsetzung stattfindet, erreicht werden.

Durch einen hohen Anfangsumsatz soll eine schnelle Absenkung der hohen, die Selektivität erniedrigenden und die Bildung von Nebenprodukten begünstigenden Acidität und Salpetersäurekonzentration in der Ausgangsmischsäure herbeigeführt werden.

Es ist aber bekannt, daß durch die Verteilung des zu nitrierenden Produktes in einer Mischsäure mit hoher Acidität und Salpetersäurekonzentration, mit der eine schnelle Weiternitrierung des gebildeten Mono-Nitroaromaten möglich ist, wie z.B. in einem Rohrreaktor ohne Rückvermischung, die Bildung unerwünschter Nebenprodukte nicht auf ein Minimum reduziert werden kann. Vor allem zu Beginn einer Nitrierung kann der bis zur Sättigungsgrenze gelöste, neu gebildete Nitrokörper (z.B. Mononitrobenzol) schnell weiternitriert oder, wie das Ausgangsprodukt, oxidativ angegriffen werden, solange die Salpetersäure noch in hohem Überschuß vorhanden und die Acidität des Säuregemisches durch das Reaktionswasser noch nicht so weit gesenkt worden ist, daß eine Weiternitrierung des gelösten Mononitroaromaten nicht mehr zu befürchten ist.

Die Verteilung der Mischsäure in Form einer hochdispersen Emulsion in den zu nitrierenden Aromaten bzw. einem Gemisch aus zu nitrierenden Aromaten und Produkt (Mononitrobenzol und Benzol), wie gleichfalls in US-A-4 973 770 vorgeschlagen, stellt ebenfalls keine Verbesserung dar, da frische, hochreaktive Mischsäure mit reduzierter Selektivität für die gewünschte einstufige Nitrierung ständig in Kontakt mit einer Organphase steht, die vor allem aus dem nitrierten Produkt, z.B. Mononitrobenzol, besteht, so daß die Bedingungen für die unerwünschte Bildung von Dinitrobenzol optimal sind.

Bei dieser Arbeitsweise besteht außerdem die Gefahr, daß, wenn mit großen Phasenverhältnissen Säurephase/Organphase von 5 - 10 : 1 wie z.B. bei einer adiabatischen Nitrierung von Benzol gearbeitet wird, in der beschriebenen Reaktionskammer die Organphase durch den hohen Überschuß an Säurephase schnell ausgewaschen wird und die Säurephase als homogene Phase vorliegt. Das führt zu vollständig unkontrollierten und undefinierten Emulgierbedingungen für die zulaufende Organphase.

Die Gefahr der Bildung großer Mengen an Nebenprodukten wird dadurch nicht reduziert, sondern erhöht.

Eine weitestgehende Unterdrückung der Bildung von unerwünschten Nebenprodukten kann nur erreicht werden, wenn die eingesetzte Mischsäure im Vergleich zum nitrierenden Produkt zu Beginn der Nitrierung im Unterschuß vorliegt und die Nitrierung in der fast vollständig ausreagierten Mischsäure (Abfallsäure) erfolgt, wie z.B. bei der Chargennitrierung von Benzol oder Toluol zu den entsprechenden Mononitroderivaten.

Bei der Chargennitrierung wird der zu nitrierende Aromat vorgelegt und die Nitriersäure wird so zugefügt, daß die Nitrierung stets in der Abfallsäure mit der niedrigst möglichen Acidität und Salpetersäurekonzentration erfolgt.

Es ist Aufgabe der vorliegenden Erfindung, ein Verfahren zum selektiven Einführen einer Nitrogruppe in einkernige Aromaten mit Gemischen aus Salpetersäure und Schwefelsäure (Mischsäure, Nitriersäure) bereitzustellen, das die Bildung unerwünschter Nebenprodukte bei der Nitrierung vermeidet.

Erfindungsgemäß wird diese Aufgabe dadurch gelöst, daß der zu nitrierende Aromat einen zentralen Treibstrahl der Mischsäure so zugeführt wird, daß er diesen umhüllt.

Als einkernige Aromaten können u.a. Benzol, Toluol, Xylole, Chlorbenzol, Dichlorbenzol, Chlortoluole, Nitrobenzol und Mononitrotoluol eingesetzt werden. Weiterhin können als einkernige Aromaten alle Aromaten eingesetzt werden, deren Nitrierprodukte bis 100 °C flüssig sind.

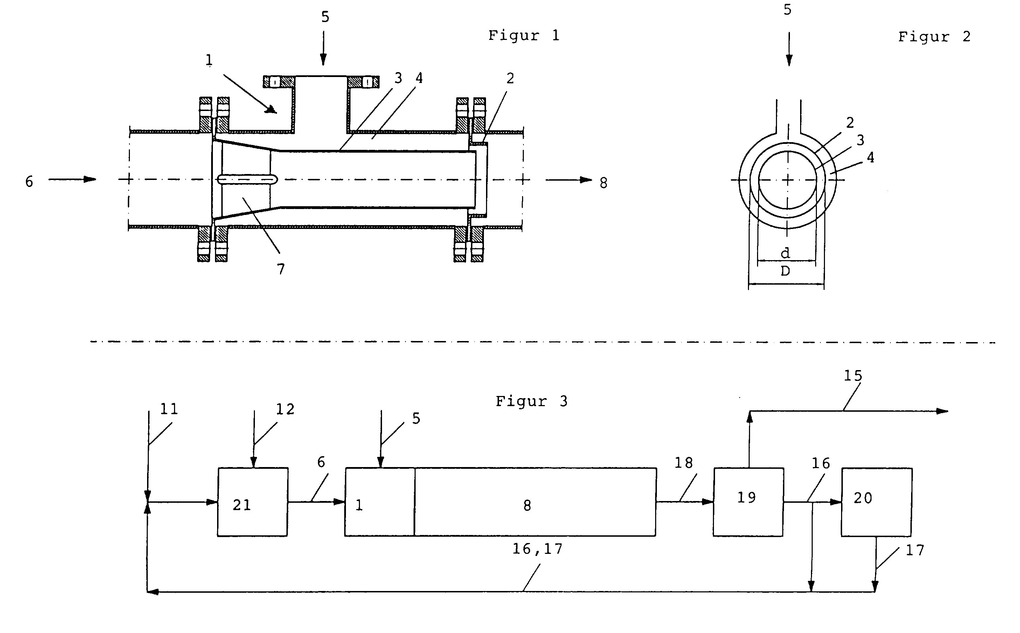

Es hat sich nämlich gezeigt, daß sich ähnliche Verhältnisse wie in der Chargennitrierung von Aromaten zu Mononitroaromaten auf diese Weise auch für eine kontinuierliche Nitrierung dieser Aromaten einstellen lassen, wenn die Zusammenmischung der Reaktionspartner derart erfolgt, daß Mischsäure als Treibstrahl eines speziell geformten Mischers (Fig. 1) mit dem zu nitrierenden Aromaten derart in Kontakt gebracht wird, daß die Mischsäure am Anfang der Nitrierung durch Mikroturbulenzen an den Grenzflächen beider Phasen in dem zu nitrierenden Aromaten nur partiell mit der optimalen Tropfengröße verteilt wird.

Damit wird erreicht, daß nur die unmittelbar mit den zu nitrierenden Aromaten in Kontakt stehenden Mischsäuren weitgehendst zu Abfallsäure ausreagiert sind und eine Weiterreaktion des gelösten Produktes, z.B. Mononitrobenzol, somit nur langsam erfolgen kann. Durch weitere Vermischung dieser bereits gebildeten Abfallsäure mit noch frischer, nicht mit nitriertem Produkt beladener Mischsäure entsteht stets eine Nitriersäure, die weitgehendst in ihrer Zusammensetzung der Abfallsäure entspricht.

Diese Vermischung von zu nitrierendem, niedrigviskosem Aromat (z.B. Benzol) mit der Nitriersäure (Mischsäure) kann in der beschriebenen Weise am besten so durchgeführt werden, daß die Nitriersäure (Säurephase) im Zentrum eines Injektors als ummantelter Freistrahl geführt wird und das zu nitrierende Produkt (Organphase) diesen zentralen Strahl umhüllt.

In der anliegenden Zeichnung zeigt schematisch
Figur 1 einen Schnitt durch die erfindungsgemäße Mischeinrichtung,
Figur 2 die Draufsicht auf die Austrittsseite der Mischeinrichtung gemäß Figur 1,
Figur 3 eine Anlage zur erfindungsgemäßen Herstellung von Nitrobenzol.

Figur 1 zeigt den erfindungsgemäßen Ringspaltmischer 1 nach Art eines modifizierten Injektors mit einem Mischrohr, das hier als Einsatzring 2 ausgebildet ist, einem Innenrohr 3, einem dazwischen liegenden Ringraum 4 mit einer Zufuhr 5 für den zu nitrierenden Aromaten, dem Eintritt 6 für die Nitriersäure sowie Leitbleche 7 im Innenrohr und ein Teil des sich anschließenden Rohrreaktors 8.

Mit einem Verhältnis der Strömungsgeschwindigkeiten V1/V2 (Säurephase zu Organphase) der beiden zu mischenden Phasen von eins und größer wird bei einem Verhältnis des Treibstrahldurchmessers (d) zum Gesamtdurchmesser des Mischrohres (D) von mindestens 0,7 bis 0,98 (Fig. 2) und Strömungsgeschwindigkeiten beider Phasen mit Reynolds-Zahlen über 5000 erreicht, daß die Säurephase unmittelbar nach dem Kontakt mit der Organphase in derselben dispergiert wird.

Das Phasenverhältnis von Mischsäure zu Aromat beträgt dabei 1 : 2 bis 10:1, und das Verhältnis der Geschwindigkeit des zentralen Treibstrahls aus Mischsäure zu der umhüllenden Aromatenphase liegt dabei zwischen 1 und 10 : 1.

Durch Leitbleche 7 im zentralen Rohr 3 oder andere Mittel wird der Mischsäure, die im Vergleich zum nitrierenden Aromaten eine höhere Dichte und Viskosität besitzt, zusätzlich zu der linearen Stromführung eine tangentiale Komponente aufgeprägt, die dazu führt, daß der zentrale Mischsäurestrahl in eine rotierende Bewegung versetzt wird. Zusätzlich kann auch der Organphasenring mit der geringeren Dichte und Viskosität im Vergleich zur Mischsäure in ähnliche Rotation gebracht werden. Dadurch wird erreicht, daß der zentrale Strahl durch die Fliehkraftwirkung noch schneller in der ihn umhüllenden Organphase und umgekehrt verteilt wird.

Durch diese Art der Zusammenführung der beiden miteinander zu mischenden Phasen wird erreicht, daß der zentrale Mischsäurestrahl von außen nach innen abreagiert, da sich zuerst nur an der Grenzfläche zwischen Säurephase und Organphase ein mit dem gebildeten Produkt gesättigte Säurephase bildet, die in ihrer Zusammensetzung der Abfallsäure entspricht.

Diese mit weiter nitrierfähigem Produkt gesättigte Grenzschicht aus "Abfallsäure" am Aromaten wird in fortschreitender Vermischung durch noch frische Mischsäure aus dem zentralen Teil des Strahles immer wieder soweit "aufgestärkt", daß eine Umsetzung möglich ist.

Eine Bildung von Nebenprodukten (z.B. Dinitrobenzol durch Weiterreaktion des zuerst gebildeten Mononitrobenzol bzw. die Bildung von Phenolen durch Oxidation des zu nitrierenden Aromaten oder des Produktes durch einen Überschuß an Salpetersäure in der Säure- oder Organphase) wird dadurch auf ein Minimum reduziert.

Besonders vorteilhaft ist diese schnelle inverse Vermischung von Säure in der Organphase zu Beginn der Nitrierung mit der Möglichkeit zur schnellen Abreaktion an den geschaffenen Grenzflächen, bei denen die Nitriergeschwindigkeit in der selben Größenordnung oder größer ist als die Mischgeschwindigkeit, wie z.B. im Falle der Nitrierung von Benzol und Toluol, bei denen die Hauptumsetzung bis 80 % oder mehr in Bruchteilen von Sekunden erfolgt, so daß bei Erreichen einer homogenen Dispersion des zu nitrierenden Aromaten und des gebildeten Nitrokörpers die Acidität und der Salpetersäuregehalt in der ursprünglichen Mischsäure und zwar in der gesamten Menge soweit abgesunken ist, daß während der für eine vollständige Umsetzung der Restsalpetersäure noch notwendigen Verweilzeit die Bildung von unerwünschten Nebenprodukten nur noch geringfügig ist.

Bevorzugt werden Mischeinrichtungen der beschriebenen Art zur Nitrierung von Aromaten wie z.B. Benzol, Toluol, Xylol und Chlorbenzol dann eingesetzt, wenn das Phasenverhältnis zwischen beiden Phasen stark von 1 abweicht, wie z.B. im Falle einer adiabatischen Nitrierung von Benzol mit einem Verhältnis Säurephase/Organphase von ca. 5 - 10 : 1, bevorzugt 7 - 8 : 1. Das gilt aber auch bei isothermer Reaktionsführung, wenn durch Rückführung der Abfallsäure oder durch Einsatz teilkonzentrierter Abfallsäuren mit einer Schwefelsäurekonzentration zwischen 85 % und 92 % das Phasenverhältnis Säurephase/Organphase groß wird.

In diesem Falle wird die im großen Überschuß vorhandene Säurephase, in der am Ende der Dispergierung die Säurephase die homogene Phase bildet, in der der zu nitrierende Aromat zusammen mit dem Produkt dispergiert ist, als zentraler Treibstrahl benutzt.

Nach dem Vermischen beider Reaktionspartner kann das Nitriergemisch zur vollständigen Umsetzung des zu nitrierenden Aromaten bzw. der noch verbliebenen Salpetersäure als Dispersion in einen Rohrreaktor ohne Rückvermischung oder auch in eine Rührkesselkaskade derart eingespeist werden, daß die im vorstehend beschriebenen Mischer erzeugte optimale Dispersion durch Turbulenz, zusätzliche statische Mischelemente oder Rühren aufrechterhalten bleibt.

Bei dem erfindungsgemäßen Verfahren wird Mischsäure einer Zusammensetzung von 20 - 30 Gew.-% Salpetersäure, 55 - 65 Gew.-% Schwefelsäure und 5 - 25 Gew.-% Wasser verwendet. In einer anderen Ausführungsform der Erfindung wird Mischsäure mit einer Zusammensetzung von 2,5 - 8,5 Gew.-% Salpetersäure, 58 - 70 Gew.-% Schwefelsäure und nicht weniger als 25 Gew.-% Wasser eingesetzt.

In einer Anlage zur Herstellung von Nitrobenzol (Fig. 3) mit einem Rohrreaktor ohne Rückvermischung als Verweilzeitstrecke wird z.B. bei einem adiabatischen Verfahren eine Mischsäure 6 der Zusammensetzung 2,5 - 8,5 % Salpetersäure, 58 - 70 % Schwefelsäure und nicht weniger als 25 % Wasser, bevorzugt eine Mischsäure der Zusammensetzung 3 - 5 % Salpetersäure und 63 - 68 % Schwefelsäure, die nach beendeter Nitrierung zu einer Abfallsäure von 62 - 71 % Schwefelsäure führt, durch Vermischen von Schwefelsäure von 68 - 72 % aus einer Aufkonzentrierung von Abfallsäure aus einer adiabatischen Nitrierung bzw. aus 96%iger Schwefelsäure 11 und zurückgeführter Abfallsäure mit 65%iger bzw. 98%iger Salpetersäure 12 in der Mischeinrichtung 21 hergestellt.

Diese Mischsäure, die entweder in Rührkesseln oder durch Inline-Mischungen einer Mischeinrichtung gemäß der Erfindung oder durch Einsatz anderer statischer Mischelemente gemischt wurde, wird auf die vorgesehene Reaktionstemperatur gebracht und als Treibstrahl für die erfindungsgemäße Mischeinrichtung benutzt.

Das zu nitrierende Benzol mit einem Überschuß von ca. 10 - 15 %, bezogen auf eingesetzte Salpetersäure, wird in einer den zentral geführten Treibstrahl 6 umschließenden Ringspaltdüse 2 mit dieser Mischsäure in der vorstehend beschriebenen Weise in Kontakt gebracht.

Durch die intensive inverse Vermischung wird der größte Teil des Benzols bereits in diesem modifizierten Injektor zum gewünschten Produkt Nitrobenzol umgesetzt. Der nachfolgende Rohrreaktor 8 ist so ausgelegt, daß die im Ringspaltmischer 1 erzeugte Dispersion erhalten bleibt und daß die Verweilzeit so groß ist, daß auch noch der Rest Salpetersäure in der weitgehend abreagierten Mischsäure verbraucht wird, d.h., das Gemisch aus dem zu nitrierenden Aromaten, dessen Nitroverbindung und der Mischsäure wird in dem rückvermischungsfreiem Rohrreaktor 8 zur endgültigen Ausnitrierung gebracht.

Das den Rohrreaktor 8 verlassende Nitriergemisch 18 wird in einem statischen oder auch dynamischen Scheider 19 getrennt. Das abgetrennte Nitrobenzol mit ca. 5 - 10 % an Benzol wird einer Wäsche zum Zwecke der Entfernung der Spuren gelöster Salpetersäure, Nitrose und Nitrophenole zugeführt und anschließend in einer Strippung/Trocknung oder Destillation vom restlichen Benzol und Wasser befreit.

Die anfallende Abfallsäure 16 wird einer Reinigung und Rekonzentrierung 20 zugeführt zur Entfernung des bei der Nitrierung gebildeten und des durch die Zugabe der Salpetersäure eingespeisten Wassers und anschließend in die Nitrierung als teilgereinigte Schwefelsäure 17 wieder zurückgeführt.

In einer alternativen Ausführungsform der Erfindung wird das Gemisch aus dem zu nitrierenden Aromaten, dessen Nitroverbindung und der Mischsäure zur endgültigen Ausnitrierung in einen Rührkessel eingebracht, wobei bereits der größte Teil des zu nitrierenden Aromaten (z.B. Benzol) zuvor in dem modifizierten Injektor zum gewünschten Mononitroprodukt umgesetzt worden ist.

Ein nach dem erfindungsgemäßen Verfahren hergestelltes Mononitrobenzol, in der das Benzol und die Mischsäure in einer erfindungsgemäßen Mischeinrichtung ineinander dispergiert wurden, enthält weniger als 200 ppm Dinitrobenzol und nicht mehr als 2000 ppm Nitrophenole (2,4-Dinitrophenol, 2,6-Dinitrophenol und Pikrinsäure). Der Pikrinsäuregehalt im Nitrophenolgemisch schwankt je nach Stärke der eingesetzten Mischsäure zwischen 10 - 50 %.

## Patentansprüche

1. Mischeinrichtung zur selektiven Nitrierung einkerniger Aromaten gekennzeichnet durch ein Mischrohr (2) mit einem Innenrohr (3), einem dazwischen liegenden Ringraum (4) mit einer Zufuhr (5) für den zu nitrierenden Aromaten und einem Eintritt (6) für die Mischsäure, wobei das Verhältnis des Durchmessers (d) des Innenrohres (3) zum Durchmesser (D) des Rohres (2) im Bereich von 0,7 bis 0,98 liegt.

2. Mischeinrichtung nach Anspruch 1, dadurch gekennzeichnet, daß in dem Innenrohr Leitbleche (7) angeordnet sind, durch die der Treibstrahl in rotierende Bewegung versetzt werden kann.

3. Verfahren zum selektiven Einführen einer Nitrogruppe in einkernige Aromaten mit Gemischen aus Salpetersäure und Schwefelsäure (Mischsäure, Nitriersäure), bei dem der zu nitrierende Aromat einem zentralen Treibstrahl der Mischsäure so zugeführt wird, daß er diesen umhüllt, dadurch gekennzeichnet, daß das Verhältnis der Geschwindigkeit des zentralen Treibstrahls aus Mischsäure zu der umhüllenden Aromatenphase 1 bis 10 : 1 beträgt.

4. Verfahren nach Anspruch 3, dadurch gekennzeichnet, daß der zentrale Treibstrahl der Mischsäure in eine rotierende Bewegung versetzt wird.

5. Verfahren nach einem der Ansprüche 3 oder 4,
dadurch gekennzeichnet, daß die Mischsäure eine Zusammensetzung von
20 bis 30 Gew.-% Salpetersäure,
55 bis 65 Gew.% Schwefelsäure
5 bis 25 Gew.-% Wasser
hat.

6. Verfahren nach einem der Ansprüche 3 oder 4,
dadurch gekennzeichnet, daß die Mischsäure eine Zusammensetzung von
2,5 bis 8,5 Gew.-% Salpetersäure,
58 bis 70 Gew.-% Schwefelsäure und
nicht weniger als 25 Gew.-% Wasser
hat.

7. Verfahren nach einem der Ansprüche 3 bis 6, wobei das Phasenverhältnis von Mischsäure zu Aromat von 1 : 2 bis 10 : 1 beträgt.

8. Verfahren nach einem der Ansprüche 1 bis 4, dadurch gekennzeichnet, daß als einkernige Aromaten Benzol, Toluol, Xylole, Chlorbenzol, Dichlorbenzol, Chlortoluole, Nitrobenzol oder Mononitrotoluol eingesetzt werden.

9. Verfahren nach einem der Ansprüche 3 bis 7, dadurch gekennzeichnet, daß das Gemisch aus dem zu nitrierenden Aromaten, dessen Nitroverbindung und der Mischsäure zur endgültigen Ausnitrierung in einen rückvermischungsfreien Rohrreaktor (8) eingeführt wird.

10. Verfahren nach einem der Ansprüche 3 bis 8, dadurch gekennzeichnet, daß das Gemisch aus dem zu nitrierenden Aromaten, dessen Nitroverbindung und der Mischsäure zur endgültigen Ausnitrierung in einen Rührkessel eingebracht wird.

## Claims

1. A mixing apparatus for the selective nitration of mononuclear aromatics, characterised by a mixing tube (2) comprising an internal tube (3), an annular space (4) located therebetween with a feed means (5) for the aromatics to be nitrated and an inlet (6) for the mixed acid, the ratio of the diameter (d) of the internal tube (3) to the diameter (D) of the tube (2) being in the range from 0.7 to 0.98.

2. A mixing apparatus according to Claim 1, characterised in that guide plates (7) are arranged in the internal tube, by means of which plates the propellant jet can be caused to move in rotating manner.

3. A process for selectively introducing a nitro group into mononuclear aromatics with mixtures of nitric acid and sulphuric acid (mixed acid, nitrating acid), in which the aromatic to be nitrated is fed to a central propellent jet of the mixed acid such that it envelops the former, characterised in that the ratio of the speed of the central propellant jet of mixed acid to the enveloping aromatic phase is 1 to 10:1.

4. A process according to Claim 3, characterised in that the central propellant jet of the mixed acid is caused to move in rotating manner.

5. A process according to one of Claims 3 or 4,
characterised in that the mixed acid has a composition of
20 to 30% by weight nitric acid,
55 to 65% by weight sulphuric acid and
5 to 25% by weight water.

6. A process according to one of Claims 3 or 4,
characterised in that the mixed acid has a composition of
2.5 to 8.5% by weight nitric acid,
58 to 70% by weight sulphuric acid and
not less than 25% by weight water.

7. A process according to one of Claims 3 to 6, wherein the phase ratio of mixed acid to aromatic is from 1:2 to 10:1.

8. A process according to one of Claims 1 to 4, characterised in that benzene, toluene, xylenes, chlorobenzene, dichlorobenzene, chlorotoluenes, nitrobenzene or mononitrotoluene are used as mononuclear aromatics.

9. A process according to one of Claims 3 to 7, characterised in that the mixture consisting of the aromatic to be nitrated, the nitro compound thereof and the mixed acid are introduced into a tubular reactor (8) which is free of back-mixing for completing nitration.

10. A process according to one of Claims 3 to 8, characterised in that the mixture consisting of the aromatic to be nitrated, the nitro compound thereof and the mixed acid are introduced into a stirred-tank reactor for completing nitration.

## Revendications

1. Dispositif de mélange pour la nitration sélective de composés aromatiques monocycliques, caractérisé par un tube mélangeur (2) avec un tube interne (3), un espace annulaire (4) situé entre ceux-ci et comprenant une arrivée (5) pour les composés aromatiques à nitrer et une entrée (6) pour le mélange sulfonitrique, le rapport du diamètre (d) du tube interne (3) au diamètre (D) du tube (2) étant compris entre 0,7 et 0,98.

2. Dispositif de mélange selon la revendication 1, caractérisé en ce que des chicanes (7) sont disposées dans le tube interne (7), au moyen desquelles le jet propulsé peut être déplacé selon un mouvement rotatif.

3. Procédé pour l'introduction sélective d'un groupe nitro dans des composés aromatiques monocycliques avec des mélanges d'acide nitrique et d'acide sulfurique (mélange sulfonitrique), dans lequel le composé aromatique à nitrer est amené dans un jet propulsé central du mélange sulfonitrique de façon à ce qu'il entoure celui-ci, caractérisé en ce que le rapport de la vitesse du jet propulsé central de mélange sulfonitrique à celle de la phase enveloppante de composé aromatique 1 est de 10 : 1.

4. Procédé selon la revendication 3, caractérisé en ce que le jet propulsé central du mélange sulfonitrique est déplacé selon un mouvement rotatif.

5. Procédé selon la revendication 3 ou 4, caractérisé en ce que le mélange sulfonitrique a une composition de 20 à 30 % en poids d'acide nitrique, 55 à 65 % en poids d'acide sulfurique et 5 à 25 % en poids d'eau.

6. Procédé selon la revendication 3 ou 4, caractérisé en ce que le mélange sulfonitrique a une composition de 2,5 à 8,5 % en poids d'acide nitrique, 58 à 70 % en poids d'acide sulfurique et pas moins de 25 % en poids d'eau.

7. Procédé selon l'une des revendications 3 à 6, dans lequel le rapport des phases du mélange sulfonitrique au composé aromatique est de 1 : 2 à 10 : 1.

8. Procédé selon l'une des revendications 1 à 4, caractérisé en ce qu'on utilise, comme composés aromatiques monocycliques, du benzène, du toluène, du xylène, du chlorobenzène, du dichlorobenzène, du chlorotoluène, du nitrobenzène ou du mononitrotoluène.

9. Procédé selon l'une des revendications 3 à 7, caractérisé en ce que le mélange formé du composé aromatique à nitrer, du composé nitro de celui-ci et du mélange sulfonitrique est amené dans un réacteur tubulaire (8) sans mélange en retour pour la nitration définitive.

10. Procédé selon l'une des revendications 3 à 8, caractérisé en ce que le mélange formé du composé aromatique à nitrer, du composé nitro de celui-ci et du mélange sulfonitrique est introduit dans une chaudière sous agitation pour la nitration définitive.
